# EUROPEAN PATENT APPLICATION

(11) **EP 3 995 453 A1**
(43) Date of publication of application: **11.05.2022**
(21) Application number: 20834786.4
(22) Date of filing: 03.07.2020
(51) Int. Cl.: C01G 23/053, C09C 1/36, C09D 17/00, A61Q 1/12, A61K 8/29

(54) **TITANIUM OXIDE POWDER, AND DISPERSION AND COSMETICS USING SAME**

(30) Priority: 03.07.2019 JP 2019124780
(71) Applicant: Sumitomo Osaka Cement Co., Ltd., Tokyo 102-8465 (JP)
(72) Inventor: YAKUBO, Teppei, Tokyo 102-8465 (JP); ITO, Naoko, Tokyo 102-8465 (JP); NEYA, Tadashi, Tokyo 102-8465 (JP)
(74) Representative: Cabinet Laurent & Charras
(86) International application number: PCT/JP2020/026211
(87) International publication number: WO 2021/002456

(57) **Abstract**

A titanium oxide powder of the present invention has a BET specific surface area of 5 m²/g or higher and 15 m²/g or lower and contains single-crystalline titanium oxide particles, in which a value (d10/d50) that is obtained by dividing a value (d10), which is obtained when a particle size distribution represented by a cumulative volume percentage of primary particle diameters of the titanium oxide particles is 10%, by a value (d50), which is obtained when a particle size distribution represented by a cumulative volume percentage thereof is 50%, is 0.3 or higher and 1 or lower, an amount of titanium oxide thereof is 99.0% by mass or more, and the titanium oxide powder has an anatase-type crystalline phase.

## Description

### Technical Field

The present invention relates to a titanium oxide powder suitable for cosmetics, and a dispersion and cosmetics using the same.

Priority is claimed on Japanese Patent Application No. 2019-124780, filed on July 3, 2019, the content of which is incorporated herein by reference.

### Background Art

In the related art, base makeup cosmetics such as foundation are widely used in order to regulate the skin to have a desired color, and to conceal pores or the like, so that the skin looks smooth. Generally, the base makeup cosmetics contains pigments in order to regulate a color of the skin. As the pigments, titanium oxide powder is frequently used.

As the titanium oxide powder used in base makeup cosmetics, for example, spindle-shaped titanium oxide particles (see, for example, Patent Literature No. 1) and octahedral-shaped titanium oxide particles (for example, see Patent Literature No. 2) are known.

### Citation List

### Patent Literatures

[Patent Literature No. 1] Japanese Laid-open Patent Publication No. H10-139434
[Patent Literature No. 2] Pamphlet of International Publication No. WO2018/003851

### Summary of Invention

### Technical Problem

However, there was a demand for titanium oxide powder to have better opacifying power in a case of being blended in cosmetics.

The present invention has been made in view of the above circumstances, and an object of the present invention is to provide a titanium oxide powder having excellent opacifying power in a case of being applied to the skin, and a dispersion and cosmetics using the same.

### Solution to Problem

That is, a titanium oxide powder of the present invention has a BET specific surface area of 5 m²/g or higher and 15 m²/g or lower and contains single-crystalline titanium oxide particles, in which a value (d10/d50) that is obtained by dividing a value (d10), which is obtained when a particle size distribution represented by a cumulative volume percentage of primary particle diameters of the titanium oxide particles is 10%, by a value (d50) , which is obtained when in a case where a particle size distribution represented by a cumulative volume percentage is 50%, is 0.3 or higher and 1 or lower, an amount of titanium oxide thereof is 99.0% by mass or more, and the titanium oxide powder has an anatase-type crystalline phase.

A dispersion of the present invention includes the titanium oxide powder of the present invention and a dispersion medium.

Cosmetics of the present invention contains the titanium oxide powder of the present invention and a cosmetic base.

### Advantageous Effects of Invention

According to the titanium oxide powder of the present invention, it is possible to provide the titanium oxide powder having excellent opacifying power in a case of being applied to the skin, and the dispersion and the cosmetics using the same.

The dispersion of the present invention is excellent in the opacifying power in a case where the cosmetics containing this dispersion is applied to the skin.

The cosmetics of the present invention is excellent in the opacifying power in a case of being applied to the skin.

### Brief Description of Drawings

FIG. 1 is a schematic diagram illustrating a state of powder and a friction sensitivity tester before an average friction coefficient is measured.
FIG. 2 is a schematic diagram illustrating a state of the powder and the friction sensitivity tester after the average friction coefficient of the powder is measured.

### Description of Embodiments

Preferred examples of embodiments of a titanium oxide powder of the present invention, and a dispersion and cosmetics using the same will be described below.

The present embodiments will be described in detail in order to better understand the gist of the invention, and the present invention is not limited unless otherwise specified. For example, unless otherwise restricted, conditions such as material, quantity, type, number, size, ratio, time, and temperature, and the like may be changed, added, or omitted as necessary.

### [Titanium Oxide Powder]

A titanium oxide powder of the present embodiment has a BET specific surface area of 5 m²/g or higher and 15 m²/g or lower. The titanium oxide powder contains single-crystalline titanium oxide particles, in which a value (d10/d50) that is obtained by dividing a value (d10), which is obtained when a particle size distribution represented by a cumulative volume percentage is 10%, by a value (d50), which is obtained when a particle size distribution represented by a cumulative volume percentage is 50%, is 0.3 or higher and 1 or lower, has an amount of titanium oxide of 99.0% by mass or more, and has an anatase-type crystalline phase.

The present inventors and the like have found that cosmetics more excellent in opacifying power can be obtained by using the above described titanium oxide powder, that is, a powder containing titanium oxide particles each of which has a predetermined size, having a uniform particle size distribution, and having an anatase-type single-crystalline phase.

### (Titanium Oxide Particles)

The titanium oxide powder of the present embodiment is an agglomerate of single-crystalline titanium oxide particles.

Since the titanium oxide powder of the present embodiment is made of single-crystalline titanium oxide particles, a refractive index of the titanium oxide powder is improved, and a light scattering property is excellent. Therefore, in a case where the titanium oxide powder is blended in cosmetics, opacifying power can be further improved.

Here, the crystallizability of the titanium oxide powder, that is, whether or not the titanium oxide particles are single-crystalline titanium oxide particles can be confirmed by the following method.

### (Crystallizability (Method of Distinguishing between single-crystalline titanium oxide particles and polycrystalline titanium oxide particles))

Whether the titanium oxide particles are single-crystalline titanium oxide particles or polycrystalline titanium oxide particles can be confirmed by the observation of a crystal axis of a particle with a field emission transmission electron microscope (FE-TEM). A single-crystalline particle is a single crystal in which a direction of the crystal axis does not change at any position of the crystal, and a polycrystalline particle is a polycrystal in which the direction of the crystal axis changes depending on a position of the crystal.

### (BET specific surface area)

A BET specific surface area of the titanium oxide powder of the present embodiment is 5 m²/g or higher and 15 m²/g or lower, and preferably 5 m²/g or higher and 13 m²/g or lower. The BET specific surface area of the titanium oxide powder of the present embodiment may be within a range of 5m²/g or higher and 8m²/g or lower, 8m²/g or higher and 10m²/g or lower, 10m²/g or higher and 13m²/g or lower, or the like.

A case where the BET specific surface area of the titanium oxide powder is 5 m²/g or higher and 15 m²/g or lower is advantageous from the viewpoint of further decreasing paleness peculiar to titanium oxide particles while having opacifying power and a feeling of transparency. In addition, in a case where the BET specific surface area of the titanium oxide powder is 5 m²/g or higher, a feeling of transparency does not deteriorate due to light scattering. On the other hand, in a case where the BET specific surface area of the titanium oxide powder is 15 m²/g or lower, scattering intensity of light having a short wavelength may not be increased as compared with scattering intensity of light having a long wavelength, and thus paleness does not increase.

As a method of measuring the BET specific surface area, for example, a method of performing measurement from a nitrogen adsorption isotherm by a BET multipoint method using a fully automated specific surface area measuring device, specifically exemplified as a fully automated specific surface area measuring device (trade name: BELSORP-Mini II, manufactured by MicrotracBEL Corp.).

### (Particle Size Distribution)

A value (d10/d50) (hereinbelow, abbreviated as "d10/d50" in some cases) that is obtained by dividing a value (d10), which is obtained when a particle size distribution represented by a cumulative volume percentage of the above described titanium oxide particles each of which has a primary particle diameter is 10%, by a value (d50), which is obtained when a particle size distribution represented by a cumulative volume percentage is 50%, is 0.3 or higher and 1 or lower. The lower limit of d10/d50 may be 0.4 or higher, or may be 0.5 or higher. The upper limit of d10/d50 may be 0.9 or lower, 0.8 or lower, 0.7 or lower, or 0.6 or lower.

Since d10/d50 is within the above described range, the cosmetics more excellent in opacifying power can be obtained in a case of being blended in the cosmetics.

d10 and d50 are obtained by, for example, the following order. A primary particle diameter of each of 50 titanium oxide particles is measured. The measured primary particle diameter is cubed and multiplied by a constant to obtain a volume. The constant may be appropriately determined according to a shape of each titanium oxide particle. For example, a constant for a polyhedral shape having eight or more sides is 0.145, and a constant for a spherical shape is 4.19 (4n/3). The measured primary particle diameters and the calculated volume values are used to calculate a volume particle size distribution of the primary particle diameters. d10 means the above described primary particle diameter in a case of a cumulative of 10%, and d50 means the above described primary particle diameter in a case of a cumulative of 50%.

Here, the primary particle diameter of the titanium oxide particle means the longest linear portion of a titanium oxide particle (longest diameter). For example, a primary particle diameter of a spherical titanium oxide particle means diameter. For example, a primary particle diameter of a rod-shaped titanium oxide particle means the length of the longest linear portion in a longitudinal direction. For example, a primary particle diameter of an octahedral-shaped titanium oxide particle means a maximum value of line segments each of which connects two apexes that face each other (hereinbelow, may be referred to as a "distance between apexes"). The two apexes that face each other are not adjacent to each other. That is, in the two apexes, the line segment that connects the apexes to each other is a line segment that does not pass through a surface of a particle but passes through the inside of the particle. The maximum value is obtained by a combination of apexes that are farthest from each other.

The primary particle diameter of the titanium oxide particle is determined by the following method. The titanium oxide powder of the present embodiment is observed with a scanning electron microscope (SEM). When observed, a predetermined number of the titanium oxide particles, for example, 200, 100, or 50 titanium oxide particles, are selected. The longest linear portion (longest diameter) of each of these titanium oxide particles is measured. By this method, a primary particle diameter of each particle can be obtained.

In a case where the titanium oxide particles are agglomerated with each other, the agglomerate particle diameter of this agglomerate is not subjected to the measurement. The titanium oxide particles (primary particles) constituting this agglomerate are measured and used as the primary particle diameters.

In a case where a shape of a titanium oxide particle is damaged, and in a case where the shape before damaged can be presumed, the primary particle diameter is measured using the shape of the particle before damaged.

Any value can be selected as d50 of the titanium oxide powder of the present embodiment as long as the BET specific surface area and d10/d50 are within the above described range, and is not particularly limited. In order to set the BET specific surface area within a predetermined range, d50 is preferably 100 nm or higher and 1000 nm or lower, more preferably 150 nm or higher and 800 nm or lower, even more preferably 200 nm or higher and 700 nm or lower, and particularly preferably 250 nm or higher and 600 nm or lower. d50 corresponds to an average primary particle diameter particles of the titanium oxide powder of the present embodiment.

### (Amount of Titanium Oxide)

The amount of titanium oxide in the present embodiment is 99.0% by mass or more. The amount of titanium oxide in the present embodiment can be obtained by measuring the titanium oxide powder of the present embodiment by a method according to "53. Titanium dioxide quantification method" described in Japanese Standards of Quasi-Drug Ingredients 2006 (JSQI).

Specifically, a sample (titanium oxide powder) is dried, about 0.2 g thereof is precisely weighed and placed in a 500 mL Erlenmeyer flask, 3 mL to 5 mL of water is added, and the mixture is shaken. Then, 30 mL of sulfuric acid and 12 g of ammonium sulfate are added, and the mixture is first heated gradually and then strongly heated to be dissolved. After cooling, 120 mL of water and 40 mL of hydrochloric acid are added while being careful that the temperature of the liquid does not rise to 50°C or higher, and shaken well to be dissolved. 3 g of metallic aluminum are added to generate hydrogen gas, a U-shaped tube with a rubber stopper is inserted, and an other end is inserted into a jar containing a sodium hydrogen carbonate solution (saturated). After the metallic aluminum is completely melted and the liquid in the above described Erlenmeyer flask turns transparent purple, the Erlenmeyer flask is allowed to cool for a while, and then the above described Erlenmeyer flask is cooled to 50°C or lower with running water. The U-shaped tube with a rubber stopper is removed, 3 mL of potassium thiocyanate solution (saturated) is added to the above described Erlenmeyer flask as an indicator, and the mixture is immediately titrated with a 0.1 mol/L ferric ammonium sulfate solution. In the titration, a point in which the light brown color of the liquid does not disappear for about 30 seconds is defined as the end point. By setting 1 mL of the 0.1 mol/L ferric ammonium sulfate solution to 7.987 mg TIO₂, the amount of titanium oxide contained in the sample (titanium oxide powder) is calculated, and the amount of titanium oxide is determined.

The amount of titanium oxide is preferably 99.1% by mass or more, more preferably 99.2% by mass or more, and even more preferably 99.3% by mass or more. The amount of titanium oxide may be 99.5% by mass or more or 99.7% by mass or more. The upper limit is 100% by mass.

Since the purity is high as the amount of titanium oxide in the titanium oxide powder is 99.0% by mass or more, light can be scattered with a small amount of addition in a case where the titanium oxide powder is blended in cosmetics. Therefore, in the case where the titanium oxide powder is blended in cosmetics, the opacifying power and feeling of transparency are excellent. The titanium oxide particles of the present embodiment contain no titanium oxide particles that are doped with a foreign element. That is, the titanium oxide powder of the present embodiment has a total amount of Ti and O of 99.0% by mass or more. The above described total amount is preferably 99.1% by mass or more, more preferably 99.2% by mass or more, and even more preferably 99.3% by mass or more.

### (Crystalline Phase)

The titanium oxide powder of the present embodiment has an anatase-type crystalline phase. A case where the titanium oxide powder has the anatase-type crystalline phase is advantageous from the viewpoints that opacifying power is further increased in a case where cosmetics containing the titanium oxide powder is applied to the skin, and a color that is close to a color of the human skin is obtained in a case where the titanium oxide powder is mixed with a cosmetic base.

The fact that the titanium oxide powder has the anatase-type can be confirmed by, for example, an X-ray diffractometer, specifically exemplified as an X-ray diffractometer (trade name: X'Pert PRO, manufactured by Spectris Co., Ltd.). In a case where a measurement result obtained by the X-ray diffractometer is an anatase single phase, the titanium oxide powder has the anatase-type.

### (Crystallinity)

The titanium oxide powder of the present embodiment preferably has a crystallinity of 0.95 or higher, more preferably 0.96 or higher, even more preferably 0.97 or higher, and particularly preferably 0.98 or higher. The upper limit of the crystallinity of the titanium oxide powder of the present embodiment is 1.0.

In the case where the crystallinity is 0.95 or higher, a refractive index of each of the titanium oxide particles is increased, scattering intensity of light is increased, and light can be scattered with a small amount of addition. Therefore, in a case where the titanium oxide powder is blended in cosmetics, the opacifying power and feeling of transparency are excellent.

The crystallinity of the titanium oxide powder can be measured by X-ray diffraction (X-ray diffraction (XRD)). Specifically, for example, first, X-ray intensity is measured in an output of 45 kV and 40 mA at a diffraction angle 2θ in a range of 20° to 30° using CuKα ray as an X-ray source with an X-ray diffractometer, preferably with an X-ray diffractometer (trade name: X'Pert PRO MPS, manufactured by Malvern Panalytical Ltd.). Profile-fitting of the obtained X-ray diffraction pattern is carried out for a crystalline portion (peak) and an amorphous portion (halo), and integrated intensity of each the portions is calculated. A ratio of the integrated intensity of the crystalline portion to a total integrated intensity is defined as a crystallinity.

### (L* value)

It is preferable that an L* value in L*a*b* color space of the titanium oxide powder of the present embodiment is 90 or higher. In a case where the L* value is 90 or higher, it is possible to suppress a phenomenon in which the color appearance of the applied color differs depending on angles at which the skin is viewed in a case of using the cosmetics.

On the other hand, in a case where the titanium oxide powder having an L* value of less than 75 is blended in the cosmetics, the appearance of the applied color is deteriorated, which is not preferable. In a case where the titanium oxide powder having an L* value of 75 or more and less than 90 is blended, the color appearance of the applied color is improved, but the appearance of the applied color differs depending on angles at which the skin is viewed, which is thus not preferable.

The higher the upper limit value of the L* value is preferable, but the upper limit may be 100, 99, 97, or 95.

An example of a method of measuring the L* value in the L*a*b* color space includes a method with a spectroscopic variable angle color difference meter (trade name: GC5000, manufactured by NIPPON DENSHOKU INDUSTRIES Co.,Ltd.).

Here, a phenomenon in which the color appearance differs depending on angles at which the skin is viewed will be described.

It is considered that the reason why the color of cosmetics such as foundation looks different depending on angles at which the skin is viewed is that the titanium oxide powder contained in the foundation cannot scatter light over a wide range.

In a case where the applied color looks different depending on angles at which the skin is viewed, the skin color that looks bright when viewed from the front, but in a case where the skin color is seen from an oblique angle, the face color may look pale, which is not preferable.

In the case of the titanium oxide powder having the above described L* value of 90 or higher, the present inventors enable the mean square displacement value described later to be 5.0 × 10⁻⁵ or lower. Therefore, the present inventors have found that angle dependence of the color appearance is low and the phenomenon in which the color appearance is different is suppressed.

### (Method of Calculating Mean Square Displacement Value)

Cosmetics that contains the titanium oxide powder and pigments are prepared. For example, an O/W type liquid foundation in which decamethylcyclopentasiloxane containing 6% by mass of the titanium oxide powder, 0.7% by mass of a yellow pigment, 0.2% by mass of a red pigment, and 0.1% by mass of a black pigment was used as a base material is produced.

This liquid foundation is applied to a glass substrate by screen printing to a thickness of about 20 µm. Leaving it to stand for 3 minutes, the glass substrate on which the liquid foundation is applied is dried with a hot plate at 200°C for 5 minutes and used as a measurement sample 1 having a thickness of about 10 µm.

By using a spectrophotometer, for example, a spectrophotometer (trade name: V-700, manufactured by JASCO Corporation), a reflectance (hereinafter, referred to as a "reflectance (1)" or an "omnidirectional reflectance (1)") of the measurement sample 1 in a wavelength range of 400 nm to 800 nm is measured with an integrating sphere. The measurement of the reflectance is carried out at an interval of 1 nm.

Next, a black spacer having a thickness of 9 mm is provided at a place other than the measurement point (a place where the incident light of the spectrophotometer does not reach) on the coated surface of the measurement sample 1, thereby obtaining a measurement sample 2. Then, similarly to the measurement sample 1, a reflectance of the measurement sample 2 in a wavelength of 400 nm to 800 nm (hereinafter, referred to as a "reflectance (2)") is measured with an integrating sphere at an interval of 1 nm.

As the reflectance (2), since light scattered at a wide angle is absorbed by the black spacer before reaching the integrating sphere, only the reflectance of light having a small scattering angle is measured.

The reflectance (1) - the reflectance (2) is calculated for each reflectance in a wavelength range of 400 nm to 800 nm to calculate a high-angular scattering reflectance (hereinafter, referred to as a "reflectance (3)" or a "high-angular reflectance (3)") in the above described range. The high-angular scattering reflectance means a reflectance having a large light scattering angle. The omnidirectional reflectance is a reflectance that includes a reflectance of light having a large scattering angle and a reflectance of light having a small scattering angle.

Next, each calculated reflectance (3) in a wavelength range of 400 nm to 800 nm is divided by each reflectance (1) in the above described range (reflectance (3)/reflectance (1)) to calculate a ratio of the high-angular scattering reflectance to the omnidirectional reflectance.

Next, an arithmetic mean value (hereinafter, referred to as an "arithmetic mean value (4)") of the ratio of the high-angular scattering reflectance in a wavelength range of 400 nm to 800 nm (reflectance (3)/reflectance (1)) is calculated.

Next, the ratio of the high-angular scattering reflectance to the omnidirectional reflectance (reflectance (3)/reflectance (1)) is divided by the arithmetic mean value (4) ((reflectance (3)/reflectance (1)/arithmetic mean value (4)), thereby obtaining a standard value (hereinafter, referred to as a "standard value (5)") at each wavelength.

Next, a value of a mean square displacement between the standard value (5) and 1 is calculated. That is, (standard value (5) - 1)² is calculated at each wavelength, and a square displacement (hereinafter referred to as a "square displacement (6)") is calculated at each wavelength. The square displacement (6) at a wavelength of 400 nm to 800 nm is arithmetically averaged, and a mean square displacement value (hereinafter, referred to as a "mean square displacement value (7)") is calculated.

The fact that the mean square displacement value (7) obtained in this way is small means that angle dependence of the color appearance of the applied color is small, and the phenomenon in which the color appearance differs depending on angles at which the skin is viewed is suppressed.

### (Average Friction Coefficient)

An average friction coefficient of the titanium oxide powder of the present embodiment, which is measured at 0.245 N per 1 cm², is preferably 0.5 or lower, more preferably 0.4 or lower, and even more preferably 0.3 or lower. A lower limit value of the average friction coefficient is 0, but may be 0.1 or more.

In a case where the average friction coefficient is 0.5 or lower, and the titanium oxide powder is blended in the cosmetics, spreadability is good, and a feeling of roughness is reduced. Therefore, the cosmetics having an excellent texture can be obtained.

The texture in the present embodiment is, for example, a feel of touching the skin to which the cosmetics is applied with the fingers when the cosmetics in which the titanium oxide powder is blended is applied to the skin.

The average friction coefficient of the titanium oxide powder of the present embodiment is a value measured by the following procedure using a friction sensitivity tester, preferably a friction sensitivity tester (model number: KES-SE, manufactured by Kato Tech Co., Ltd.).

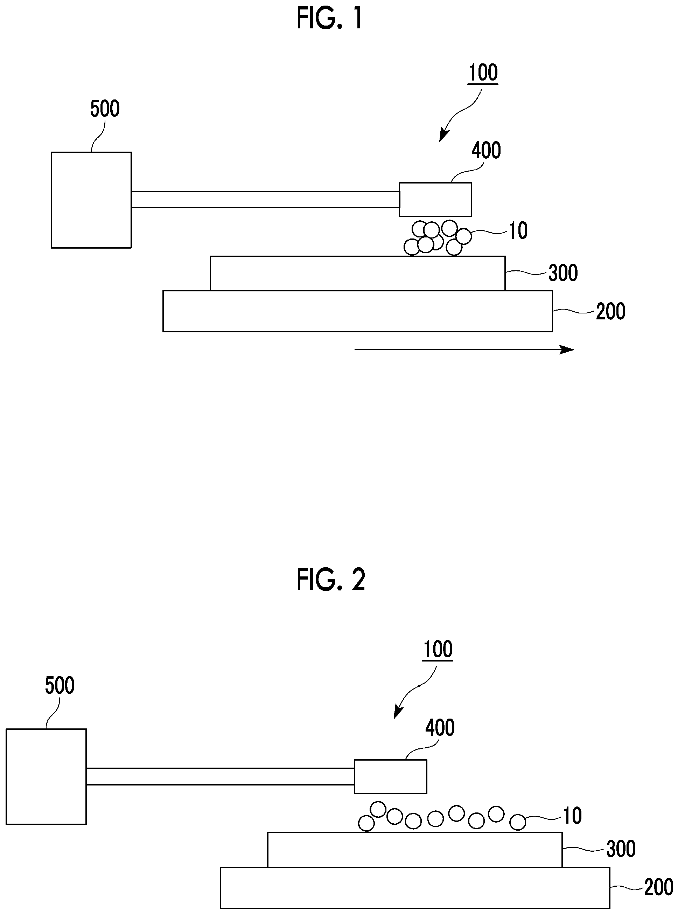

FIG. 1 is a schematic diagram illustrating a state of a friction sensitivity tester and a powder before measuring the average friction coefficient.

As illustrated in FIG. 1, a friction sensitivity tester 100 is provided with a stage 200, a skin model substrate 300 disposed on the stage 200, a piano wire sensor 400 that measures a friction coefficient of a titanium oxide powder 10 disposed on the skin model substrate 300, and a detector 500 that detects the friction coefficient measured by the piano wire sensor 400.

As illustrated in FIG. 1, 1 g of the titanium oxide powder 10 is placed on the skin model substrate (example of substrate: cheek skin model 30s (ϕ55 mm × 5 Tmm), manufactured by Beaulax Co., Ltd.) 300 disposed on the stage 200, and this skin model substrate is disposed so that the titanium oxide powder 10 is below the 1 cm² piano wire sensor (device standard) 400. The entire titanium oxide powder 10 is required to be placed below the piano wire sensor 400. Therefore, the titanium oxide powder 10 is disposed on the skin model substrate 300 within a range of 1 cm². Next, the piano wire sensor 400 is brought into contact with the titanium oxide powder 10 so as to have a load of 25 g (25 × 10⁻³ kg × 9.8 m/s² = 0.245 N). Next, the skin model substrate 300 is horizontally moved by 30 mm at a speed of 1 mm/s on the stage 200, so that average friction coefficient of the titanium oxide powder 10 can be measured.

FIG. 2 is a schematic diagram illustrating a state after the skin model substrate on which the titanium oxide powder is placed is horizontally moved on the stage.

A secondary particle diameter of the titanium oxide powder of the present embodiment is preferably 1 µm or higher and 10 µm or lower, more preferably 2 µm or higher and 9 µm or lower, even more preferably 3 µm or higher and 8 µm or lower, particularly preferably 4 µm or higher and 8 µm or lower.

In a case where an average particle diameter of the secondary particles is 1 µm or higher and 10 µm or lower, it is preferable that a feeling of roughness is suppressed when the cosmetics in which the titanium oxide powder is blended is applied to the skin and the texture is excellent.

In a case where the average particle diameter of the secondary particles is 1 µm or higher, the average primary particle diameter does not become too small, and the opacifying power and the feeling of transparency can be obtained, which is preferable. On the other hand, in a case where the average particle diameter of the secondary particles is 10 µm or lower, there is no feeling of roughness when the cosmetics in which the titanium oxide powder is blended is applied to the skin, and the texture not deteriorate, which is preferable.

The "average secondary particle diameter " of the titanium oxide particles of the present embodiment is a numerical value obtained by the following method. That is, the titanium oxide powder of the present embodiment is dry-measured using a particle size distribution measuring device, preferably a particle size distribution measuring device MASTERSIDER 3000 (manufactured by Malvern Panalytical Ltd.). A particle diameter (d50) in a case where the obtained volume-based particle size distribution represented by a cumulative volume percentage is 50% is the average secondary particle diameter of the present embodiment.

### (Surface Treatment)

The titanium oxide powder of the present embodiment may have any of an inorganic compound or an organic compound on a surface thereof.

Examples of a method of attaching any of the inorganic compound or the organic compound to the surface of each of the titanium oxide particles include a method of performing a surface treatment with a surface treatment agent, and the like.

The surface treatment agent is not particularly limited as long as the surface treatment agent can be used in cosmetics, and can be appropriately selected depending on a purpose. Examples of the surface treatment agent include an inorganic component, an organic component, and the like.

Examples of the inorganic component include inorganic oxide. Examples thereof include silica, alumina and the like.

Examples of the organic component include a silicone compound, organopolysiloxane, a fatty acid, fatty acid soap, fatty acid ester, an organic titanate compound, a surfactant, a non-silicone compound, and the like. One of these organic components may be used alone, or two or more thereof may be used in combination.

Examples of the silicone compound include silicone oils such as methylhydrogenpolysiloxane, dimethylpolysiloxane, and methylphenylpolysiloxane; alkylsilanes such as methyltrimethoxysilane, ethyltrimethoxysilane, hexyltrimethoxysilane, and octyltrimethoxysilane; fluoroalkylsilanes such as trifluoromethylethyltrimethoxysilane and heptadecafluorodecyltrimethoxysilane; methicone, hydrogendimethicone, triethoxysilylethylpolydimethylsiloxyethyldimethicone, triethoxysilylethylpolydimethylsiloxyethylhexyldimethicone, (acrylate/tridecylic acrylate/triethoxysilylpropyl methacrylate/dimethicone methacrylate) copolymers, and triethoxycaprylylsilane. In addition, the silicone compound may be a monomer of a compound or a copolymer thereof. One of these silicone compounds may be used alone, or two or more thereof may be used in combination.

As the fatty acid, for example, palmitic acid, isostearic acid, stearic acid, lauric acid, myristic acid, behenic acid, oleic acid, rosin acid, 12-hydroxystearic acid, and the like are mentioned.

As the fatty acid soap, for example, aluminum stearate, calcium stearate, aluminum 12-hydroxystearate, and the like are mentioned.

As the fatty acid ester, for example, dextrin fatty acid ester, cholesterol fatty acid ester, sucrose fatty acid ester, starch fatty acid ester, and the like are mentioned.

As the organic titanate compound, for example, isopropyl triisostearoyl titanate, isopropyl dimethacryl isostearoyl titanate, isopropyl tri(dodecyl) benzene sulfonyl titanate, neopentyl (diallyl)oxy-tri(dioctyl) phosphate titanate, neopentyl (diallyl)oxy-trineododecanoyl titanate, and the like are mentioned.

According to the titanium oxide powder of the present embodiment, in a case where the cosmetics containing the titanium oxide powder is applied to the skin, opacifying power is excellent. According to the titanium oxide powder of the present embodiment, in the case where cosmetics containing the titanium oxide powder is applied to the skin, it is possible to obtain natural finish in which paleness peculiar to the titanium oxide particles is decreased and an excellent feeling of transparency is achieved in addition to excellent opacifying power.

### [Method of Producing Titanium Oxide Powder]

A method of producing a titanium oxide powder of the present embodiment includes a first step of preparing a reaction solution by mixing a hydrolyzed product of a titanium alkoxide or a hydrolyzed product of a titanium metal salt with a compound having a five-membered ring that contains nitrogen, and subjecting this reaction solution to hydrothermal synthesis, to produce titanium oxide particles.

In addition, the method of producing a titanium oxide powder of the present embodiment includes, as necessary, a second step of mixing a reaction solution containing the titanium oxide particles after the hydrothermal synthesis obtained in the first step with the same reaction solution in the first step before the hydrothermal synthesis, and performing hydrothermal synthesis.

In addition, the method of producing a titanium oxide powder of the present embodiment includes a third step of cleaning the reaction solution obtained in the first step or the second step and calcining the reaction solution at 650°C or higher and 850°C or lower to remove organic substances.

In addition, the method of producing a titanium oxide powder of the present embodiment includes a fourth step of cracking the titanium oxide powder obtained in the third step.

Each step will be described below.

### (First Step)

The first step is a step of producing titanium oxide particles.

In the first step, the hydrolyzed product of the titanium alkoxide or the hydrolyzed product of the titanium metal salt is mixed with the compound having a five-membered ring that contains nitrogen to prepare the reaction solution (slurry), and this reaction solution is subjected to hydrothermal synthesis to produce titanium oxide particles.

Materials used in the first step will be described below.

### (Hydrolyzed Product of Titanium Alkoxide or Hydrolyzed Product of Titanium Metal Salt)

The hydrolyzed product of titanium alkoxide or the hydrolyzed product of titanium metal salt is obtained by hydrolyzing the titanium alkoxide or the titanium metal salt.

The hydrolyzed product is, for example, a cake-like solid which is a white solid, and is hydrated titanium oxide called metatitanic acid or orthotitanic acid.

As titanium alkoxide, for example, tetraethoxytitanium, tetraisopropoxytitanium, tetra-n-propoxytitanium, tetra-n-butoxytitanium, and the like are mentioned. One of these titanium alkoxides may be used alone, or two or more thereof may be used in combination. Among these, tetraisopropoxytitanium and tetra-n-butoxytitanium are preferable, and tetraisopropoxytitanium is more preferable, from the viewpoint of easy availability and easy control of a hydrolysis rate.

Examples of the titanium metal salt include titanium tetrachloride, titanium sulfate, and the like. One of these titanium metal salts may be used alone, or two or more thereof may be used in combination.

In the present embodiment, in order to obtain high purity anatase-type titanium oxide particles, it is preferable to use a high purity titanium alkoxide or a high purity titanium metal salt.

The hydrolyzed product contains a by-product such as alcohols, hydrochloric acid, and sulfuric acid.

Since the by-product inhibits nucleation and crystal growth of titanium oxide particles, it is preferable to clean the hydrolyzed product with pure water in advance.

Examples of a method of cleaning the hydrolyzed product include decantation, Nutsche method, ultrafiltration method, and the like.

### (Compound Having Five-membered Ring That Contains Nitrogen)

The compound having a five-membered ring that contains nitrogen is contained in the reaction solution due to a function as a pH adjuster of the reaction solution and a function as a catalyst for hydrothermal synthesis.

Examples of the compound having a five-membered ring that contains nitrogen include pyrrole, imidazole, indole, purine, pyrrolidine, pyrazole, triazole, tetrazole, isothiazole, isoxazole, furazan, carbazole, 1,5-diazabicyclo-[4.3.0]-5-nonene, and the like. One of these compounds having a five-membered ring that contains nitrogen may be used alone, or two or more thereof may be used in combination.

Among these, as the compound having a five-membered ring that contains nitrogen, a compound containing one nitrogen atom is preferable from the viewpoint of narrowing a particle size distribution of the titanium oxide powder and further improving crystallizability. For example, pyrrole, indole, pyrrolidine, isothiazole, isoxazole, furazan, carbazole, 1,5-diazabicyclo-[4.3.0]-5-nonene, and the like are preferable.

Among these, as the compound having a five-membered ring that contains nitrogen, a compound which contains one nitrogen atom and of which a five-membered ring has a saturated heterocyclic structure is more preferable from the viewpoint of narrowing a particle size distribution of the titanium oxide powder and further improving crystallizability. For example, pyrrolidine, and 1,5-diazabicyclo-[4.3.0]-5-nonene are more preferable.

### (Reaction Solution)

A method of preparing the reaction solution is not particularly limited, and can be appropriately selected depending on a purpose. For example, a method of mixing by using a stirrer, a bead mill, a ball mill, an attritor, a dissolver, or the like, and the like are mentioned.

In addition, water may be added to the reaction solution to adjust a concentration of the reaction solution. Examples of the water to be added to the reaction solution include deionized water, distilled water, pure water, and the like.

The pH of the reaction solution is preferably 9 or higher and 13 or lower, and more preferably 11 or higher and 13 or lower, from the viewpoint that a catalytic action of the compound having a five-membered ring that contains nitrogen appropriately functions and a nucleation rate becomes appropriate.

In a case where the pH of the reaction solution is in a range of 9 or higher and 13 or lower, production of the titanium oxide particles and efficiency of crystal growth become better.

The pH of the reaction solution can be regulated by controlling the amount of the compound having a five-membered ring that contains nitrogen.

In order to obtain the titanium oxide powder of the present embodiment, a concentration of titanium atoms in the reaction solution is preferably 1.25 mol/L or more and 3.0 mol/L or less, and more preferably 1.5 mol/L or more and 2.5 mol/L or less.

In other words, a concentration of a titanium oxide content in the reaction solution is preferably 10% by mass or more and 24% by mass or less, and preferably 12% by mass or more and 20% by mass or less. The titanium oxide content means the mass of titanium oxide produced from titanium alkoxide or a titanium metal salt used as a raw material. For example, in a case where 1 mol of tetraethoxytitanium is used, 1 mol of titanium oxide is produced, so that the titanium oxide content of 1 mol of tetraethoxytitanium is the mass of 1 mol of titanium oxide, that is, 80 g.

Since in the case where the concentration of titanium atoms in the reaction solution is 1.25 mol/L or more and 3.0 mol/L or less, a nucleation rate becomes appropriate, production of titanium oxide particles and efficiency of crystal growth become better.

The concentration of titanium atoms in the reaction solution can be regulated by controlling the amount of a hydrolyzed product of titanium alkoxide or a hydrolyzed product of titanium metal salt.

A molar ratio (titanium atom:compound having a five-membered ring that contains nitrogen) of titanium atoms to compounds having a five-membered ring that contains nitrogen in the reaction solution is preferably 1.0:0.5 to 1.0:2.0, and more preferably 1.0:0.6 to 1.0:1.8, and even more preferably 1.0:0.7 to 1.0:1.5.

In a case where the molar ratio of titanium atoms to compounds having a five-membered ring that contains nitrogen in the reaction solution is within the above described range, it is possible to produce a titanium oxide powder that has the higher light scattering property and contains octahedral-shaped titanium oxide particles.

### (Hydrothermal Synthesis)

Hydrothermal synthesis is a method in which a reaction solution is heated to allow titanium in the reaction solution to react in the presence of high-temperature and high-pressure hot water.

The hydrothermal synthesis is carried out by placing the reaction solution in a high-temperature and high-pressure container called an autoclave, sealing the autoclave, and heating the reaction solution together with the autoclave.

In a case where the reaction solution is heated, a pressure in the container rises due to evaporation of moisture in the reaction solution, which allows a high-temperature and high-pressure reaction to occur.

In the hydrothermal synthesis in the first step, the titanium oxide particles are produced by holding the reaction solution at two different temperatures for a predetermined time. In the hydrothermal synthesis in the first step, the temperatures at which the reaction solution is held in a heated state are referred to as heating and holding temperatures. In addition, among the heating and holding temperatures, a low temperature is referred to as a first heating and holding temperature, and a high temperature is referred to as a second heating and holding temperature. In addition, the time for holding the first heating and holding temperature is referred to as a first heating and holding time, and the time for holding the second heating and holding temperature is referred to as a second heating and holding time.

Any temperature can be selected as the first heating and holding temperature in the hydrothermal synthesis, but the first heating and holding temperature is preferably 100°C or higher and 200°C or lower, and more preferably 120°C or higher and 180°C or lower.

By carrying out the second heating in the hydrothermal synthesis, the production and the growth reaction of particles can be separated, and particles having a high degree of crystallinity can be obtained. That is, in the first heating, it is considered that new fine particles are produced together with the growth of the generated fine particles since the temperature continuously increases, so that coalescence of the fine particles that have not sufficiently grown occurs. The crystallizability of the particles produced by the coalescence of the fine particles is lowered due to the presence of interface misfit or the like between the fine particles. In a case where the first heating and holding temperature in the hydrothermal synthesis is within the above described range, it is possible to prevent the titanium oxide fine particles from being excessively produced.

Any time can be selected as the first heating and holding time in the hydrothermal synthesis, but the first heating and holding time is preferably 1 hour or longer and 7 hours or shorter, more preferably 1 hour or longer and 6 hours or shorter, and even more preferably 2 hours or longer and 5 hours or shorter.

In a case where the first heating and holding time in the hydrothermal synthesis is within the above described range, the produced titanium oxide fine particles can grow to consume the raw material, so that the production of new fine particles during the second heating and holding can be prevented.

Any temperature can be selected as the second heating and holding temperature in the hydrothermal synthesis, but the second heating and holding temperature is preferably 200°C or higher and 350°C or lower, and more preferably 200°C or higher and 300°C or lower.

In a case where the second heating and holding temperature in the hydrothermal synthesis is within the above range, the growth reaction of the produced titanium oxide particles efficiently occurs.

Any time can be selected as the second heating and holding time in the hydrothermal synthesis, but the second heating and holding time is preferably 1 hour or longer and 24 hours or shorter, and more preferably 2 hours or longer and 12 hours or shorter.

In a case where the second heating and holding time in the hydrothermal synthesis is within the above described range, the titanium oxide particles sufficiently grow and are excellent in production efficiency.

A heating rate in the hydrothermal synthesis is not particularly limited, and can be appropriately selected depending on a purpose.

A pressure in the hydrothermal synthesis is a pressure in a case where the reaction solution is heated to the above described temperature range in a high-temperature and high-pressure container.

During heating in the autoclave, it is preferable to stir the reaction solution using a stirring device.

A stirring speed is not particularly limited, can be appropriately selected depending on a purpose, and is preferably 100 rpm or higher and 300 rpm or lower.

### (Second Step)

A second step is a step of growing crystals of the titanium oxide particles obtained in the first step. The second step is carried out in a case where the size of each of the titanium oxide particles obtained is smaller than a desired size.

The second step is a step of mixing the reaction solution containing the titanium oxide particles after the hydrothermal synthesis obtained in the first step with the reaction solutions (the hydrolyzed product of titanium alkoxide or the hydrolyzed product of titanium metal salt, and the compound having a five-membered ring that contains nitrogen) prepared in the first step before the hydrothermal synthesis to carry out hydrothermal synthesis. The step of mixing the reaction solution provided in the first step before the hydrothermal synthesis may be carried out by a method of adding the hydrolyzed product of titanium alkoxide or the hydrolyzed product of titanium metal salt, and the compound having a five-membered ring that contains nitrogen to the reaction solution containing the titanium oxide particles after the hydrothermal synthesis, separately.

A mixing ratio of the reaction solution containing the titanium oxide particles after the hydrothermal synthesis obtained in the first step to the reaction solutions (the hydrolyzed product of titanium alkoxide or the hydrolyzed product of titanium metal salt, and the compound having a five-membered ring that contains nitrogen) prepared in the first step is preferably 1:1 to 1:20 in terms of the mass of the titanium oxide particles. The ratio may be 1:1 to 1:5, 1:5 to 1:10, 1:10 to 1:20, or the like, as necessary.

The hydrothermal synthesis in the second step can be performed under the same conditions as in the first step.

### (Third Step)

A third step is a step of cleaning and calcining the reaction solution obtained in the first step or the second step, and removing organic substances. The third step is carried out to remove organic substances remaining in the reaction solution.

In the cleaning step, an acid is added to the reaction solution obtained in the first step or the second step until the pH reaches 7. Then, the reaction solution adjusted to pH 7 is cleaned with pure water until conductivity of a filtrate is 100 µS/cm or lower.

The acid is not particularly limited as long as an acid can neutralize the reaction solution and does not remain in the titanium oxide powder after a calcining step described later. For example, an inorganic acid can be used, and specifically, hydrochloric acid, nitric acid, sulfuric acid, or the like can be used.

After cleaning, a solid matter containing the titanium oxide particles is collected and dried. Examples of the method of taking out the titanium oxide particles from the solution include a method of carrying out solid-liquid separation such as decantation and Nutsche method, and the like.

The solid matter containing titanium oxide particles taken out by solid-liquid separation may be naturally dried, or may be dried at a drying temperature higher than room temperature, for example, 150°C to 400°C.

Next, the obtained solid matter is calcined at 650°C or higher and 850°C or lower. The organic substances are removed by the calcination in the above described range while maintaining the shape of the titanium oxide particles, and the titanium oxide powder of the present embodiment, which has an L* value of 90 or higher can be obtained.

The obtained titanium oxide powder can be stored by a preferred method selected as necessary.

### (Fourth Step)

In a fourth step, the titanium oxide powder obtained in the third step is subjected to cracking so that an average secondary particle diameter is 1 µm or higher and 10 µm or lower. The cracking method is not particularly limited, and examples thereof include a method of cracking titanium oxide powder with a known crusher. Examples of the crusher include a pin mill, a hammer mill, a jet mill, an impeller mill, and the like.

The titanium oxide powder after the organic substances are removed is taken out and subjected to cracking, thereby capable of obtaining the titanium oxide powder of the present embodiment.

In the present embodiment, the method of cracking the titanium oxide powder after drying is exemplified, but the titanium oxide powder of the present embodiment can also be obtained by a wet cracking and drying method before the drying is carried out.

### (Surface Treatment)

It is also possible to subject the titanium oxide particles to a surface treatment. A timing of carrying out the surface treatment is not particularly limited, and can be appropriately selected depending on a purpose. As the timing of carrying out the surface treatment, for example, after the second step, after the third step, after the fourth step, and the like are mentioned.

A method of carrying out a surface treatment is not particularly limited, and known methods can be appropriately selected depending on types of surface treatment agents to be used.

### [Dispersion]

The dispersion of the present embodiment contains the titanium oxide powder of the present embodiment and a dispersion medium. The dispersion of the present embodiment contains other components as necessary.

The dispersion of the present embodiment may be in a low-viscosity liquid state or a high-viscosity paste state.

The amount of the titanium oxide powder in the dispersion of the present embodiment is not particularly limited, and can be appropriately selected depending on a purpose.

### (Dispersion Medium)

The dispersion medium is not particularly limited as long as the dispersion medium can be blended with cosmetics, and can be appropriately selected depending on a purpose. Examples of the dispersion medium include water, alcohols, esters, ethers, ketones, hydrocarbon, amides, polysiloxanes, modified polysiloxanes, hydrocarbon oil, ester oil, a higher fatty acid, higher alcohol, and the like. One of these dispersion media may be used alone, or two or more thereof may be used in combination.

Examples of the alcohols include methanol, ethanol, 1-propanol, 2-propanol, 1-butanol, 2-butanol, octanol, glycerin, and the like.

Examples of the esters include ethyl acetate, butyl acetate, ethyl lactate, propylene glycol monomethyl ether acetate, propylene glycol monoethyl ether acetate, g-butyrolactone, and the like.

Examples of the ethers include diethyl ether, ethylene glycol monomethyl ether (methyl cellosolve), ethylene glycol monoethyl ether (ethyl cellosolve), ethylene glycol monobutyl ether (butyl cellosolve), diethylene glycol monomethyl ether, diethylene glycol monoethyl ether, and the like.

Examples of the ketones include acetone, methyl ethyl ketone, methyl isobutyl ketone, acetyl acetone, cyclohexanone, and the like.

Examples of the hydrocarbon include aromatic hydrocarbon such as benzene, toluene, xylene, and ethylbenzene; and cyclic hydrocarbon such as cyclohexane.

Examples of the amides include dimethylformamide, N,N-dimethylacetoacetamide, N-methylpyrrolidone, and the like.

Examples of the polysiloxanes include chain polysiloxanes such as dimethylpolysiloxane, methylphenylpolysiloxane, and diphenylpolysiloxane; cyclic polysiloxanes such as octamethylcyclotetrasiloxane, decamethylcyclopentasiloxane, and dodecamethylcyclohexasiloxane, and the like.

Examples of the modified polysiloxanes include an amino-modified polysiloxane, a polyether-modified polysiloxane, an alkyl-modified polysiloxane, a fluorine-modified polysiloxane, and the like.

Examples of the hydrocarbon oil include liquid paraffin, squalane, isoparaffin, branched chain light paraffin, petrolatum, ceresin, and the like.

Examples of the ester oil include isopropyl myristate, cetyl isooctanoate, glyceryl trioctanoate, and the like.

Examples of the higher fatty acid include lauric acid, myristic acid, palmitic acid, stearic acid, and the like.

Examples of the higher alcohol include lauryl alcohol, cetyl alcohol, stearyl alcohol, hexyl dodecanol, isostearyl alcohol, and the like.

### (Other Components)

The other components are not particularly limited as long as the components do not impair an effect of the dispersion of the present embodiment, and can be appropriately selected depending on a purpose.

Examples of the other components include a dispersant, a stabilizer, a water-soluble binder, a thickener, an oil-soluble preservative, an ultraviolet absorber, an oil-soluble agent, oil-soluble coloring matters, oil-soluble proteins, a vegetable oil, an animal oil, and the like. One of these components may be used alone, or two or more thereof may be used in combination.

The amount of the dispersion medium in the dispersion is not particularly limited, and can be appropriately selected depending on a purpose. The amount of the dispersion medium is preferably 10% by mass or more and 99% by mass or less, more preferably 20% by mass or more and 90% by mass or less, and even more preferably 30% by mass or more and 80% by mass or less, with respect to a total amount of the dispersion of the present embodiment.

According to the dispersion of the present embodiment, in a case where the cosmetics containing the dispersion of the present embodiment is applied to the skin, opacifying power is excellent. According to the dispersion of the present embodiment, in the case where cosmetics containing the titanium oxide powder is applied to the skin, it is possible to obtain natural finish in which paleness peculiar to titanium oxide particles is decreased and an excellent feeling of transparency is achieved in addition to excellent opacifying power.

### [Method of Producing Dispersion]

A method of producing the dispersion of the present embodiment is not particularly limited, and a known method can be adopted. Examples of the method of producing the dispersion of the present embodiment include a method of producing a dispersion by mechanically dispersing the titanium oxide powder of the present embodiment with respect to a dispersion medium by a dispersing device, and the like.

Examples of the dispersing device include a stirrer, a self-revolution type mixer, a homomixer, an ultrasonic homogenizer, a sand mill, a ball mill, a roll mill, and the like.

In a case of being applied to the skin, the dispersion of the present embodiment can decrease paleness peculiar to titanium oxide while achieving both the opacifying power and feeling of transparency.

### [Cosmetics]

The cosmetics of the present embodiment contains the titanium oxide powder of the present embodiment and a cosmetic base. In addition, cosmetics of an other embodiment contains the dispersion and a cosmetic base containing the titanium oxide of the present embodiment. The cosmetics of the present embodiment contains other components as necessary.

The amount of the titanium oxide powder in the cosmetics can be optionally selected, but the amount of the titanium oxide powder is preferably 0.1% by mass or more and 50% by mass or less, with respect to a total of the cosmetics.

### (Cosmetic Base)

The cosmetic base can be appropriately selected from those generally used for cosmetics, and examples thereof include talc, mica, and the like. One of these cosmetic bases may be used alone, or two or more thereof may be used in combination.

The amount of the cosmetic base in the cosmetics is not particularly limited, and can be appropriately selected depending on a purpose.

### (Other Components)

In addition to the titanium oxide powder and the cosmetic base of the present embodiment, the cosmetics of the present embodiment can contain other components within a range which does not impair an effect of the present embodiment.

The other components can be appropriately selected from components generally used in cosmetics. Examples of the other components include a solvent, an oil agent, a surfactant, a humectant, an organic ultraviolet absorber, an antioxidant, a thickener, a fragrance, a colorant, a physiologically active component, an antibacterial agent, and the like. One of these components may be used alone, or two or more thereof may be used in combination.

The amount of the other components in the cosmetics is not particularly limited, and can be appropriately selected depending on a purpose.

A method of producing the cosmetics of the present embodiment is not particularly limited, and can be appropriately selected depending on a purpose. Examples of the method of producing the cosmetics of the present embodiment include a producing method in which the titanium oxide powder is mixed with the cosmetic base and the mixture is mixed with the other components, a producing method in which the titanium oxide powder is mixed with existing cosmetics, a producing method in which the dispersion that contains titanium oxide is mixed with the cosmetic base and the mixture is mixed with the other components, and a producing method in which the dispersion that contains titanium oxide is mixed with existing cosmetics, and the like are mentioned.

### (Form)

A form of the cosmetics of the present embodiment is not particularly limited, and can be appropriately selected depending on a purpose. Examples of the form of the cosmetics of the present embodiment, for example, a powder-like form, powdery solid-like form, a solid-like form, a liquid-like form, a gel-like form, and the like. In a case where the form of the cosmetics is liquid-like or gel-like, a dispersion form of the cosmetics is not particularly limited, and can be appropriately selected depending on a purpose. Examples of the dispersion form of the gel-like cosmetics include a water-in-oil type (W/O type) emulsion, an oil-in-water type (O/W type) emulsion, an oil type, and the like.

Examples of the cosmetics of the present embodiment include base makeup, nail polish, lipstick, and the like. Among these, the base makeup is preferable.

Examples of the base makeup include makeup base used mainly for decreasing irregularities of the skin, foundation used mainly for adjusting a color of the skin, face powder used mainly for improving fixation of foundation to the skin, and the like.

According to the cosmetics of the present embodiment, in a case of being applied to the skin, the opacifying power is excellent. In addition, according to the cosmetics of the present embodiment, it is possible to decrease paleness peculiar to titanium oxide particles while having a feeling of transparency.

### Examples

Hereinafter, the present invention will be described more specifically with reference to Examples and Comparative Examples, but the present invention is not limited to the following Examples.

### [Example 1]

### (Production of Titanium Oxide Powder)

1 L of pure water was placed in a glass container having a capacity of 2 L, and 2 mol of tetraisopropoxytitanium (trade name: A-1, manufactured by Nippon Soda Co., Ltd.) was added dropwise while performing stirring to obtain a white suspension containing a hydrolyzed product of titanium alkoxide.

Next, the white suspension was subjected to filtration, to obtain a white cake A that is a solid portion of the hydrolyzed product of titanium alkoxide.

Next, 2 mol (160 g) of the white cake (A) in terms of titanium oxide, pyrrolidine (manufactured by Kanto Chemical Co., Inc.) in an amount of 1.4 mol, and pure water were added to an autoclave to prepare a slurry (B1) in a total amount of 1 kg. A concentration of a titanium oxide content in the slurry (B1) was 16% by mass.

Next, by using the autoclave, the slurry (B1) was held at 150°C for 6 hours, then heated to 260°C, and held at the temperature of 260°C for 6 hours to obtain a titanium oxide particle suspension (C1). That is, hydrothermal synthesis was carried out to obtain the suspension (C1).

The obtained titanium oxide particle suspension (C1) was subjected to solid-liquid separation, and the solid was dried at 200°C to obtain a titanium oxide powder of Example 1.

### (Production of Sample for Evaluation of Optical Properties)

6 g of the titanium oxide powder of Example 1, 3 g of ethyl cellulose, and 91 g of terpioneol were kneaded with a three-roll mill to produce a sample for an evaluation of optical properties of Example 1.

### (Production of Sample for Evaluation of Sensory)

2 g of the titanium oxide powder of Example 1 and 8 g of talc were mixed with each other in a mortar to produce a sample for an evaluation of sensory of Example 1.

### (Production of Foundation)

An O/W type liquid foundation that contains 6% by mass of the titanium oxide powder of Example 1, 0.7% by mass of yellow pigment, 0.2% by mass of red pigment, and 0.1% by mass of black pigment and uses decamethylcyclopentasiloxane as a base material was produced.

### [Example 2]

### (Production of Titanium Oxide Powder)

35% hydrochloric acid (manufactured by Kanto Chemical Co., Inc., special grade reagent) was added dropwise to the reaction solution (C1) obtained in the production process of Example 1 until the pH reached 7.

Next, the reaction solution was filtered, and cleaning with pure water was repeated until conductivity of a filtrate was 100 µS/cm or less.

Next, a solid matter on filter paper was collected, dried at 200°C, and then calcined at 700°C to obtain a titanium oxide powder.

The obtained titanium oxide powder was subjected to cracking with a mill (manufactured by Oster, 16 speed) to obtain a titanium oxide powder of Example 2.

### (Production of Cosmetics and the like)

In the same manner as in Example 1, a sample for an evaluation of optical properties, a sample for an evaluation of sensory, and a foundation of Example 2 were produced, respectively.

### [Example 3]

### (Production of Titanium Oxide Powder)

A titanium oxide particle suspension (C2) was obtained in the same manner as in Example 1, except that by using an autoclave, the slurry (B1) was held at 170°C for 6 hours, then heated to 260°C, and held at the temperature of 260°C for 6 hours.

35% hydrochloric acid was added dropwise to the obtained titanium oxide particle suspension (C2) until the pH reached 7 in the same manner as in Example 2, and cleaning with pure water was repeated until the conductivity of a filtrate was 100 µS/cm.

Next, a solid matter on filter paper was collected, dried at 200°C in the same manner as in Example 2, calcined at 700°C, and subjected to cracking to obtain a titanium oxide powder of Example 3.

### (Production of Cosmetics and the like)

In the same manner as in Example 1, a sample for an evaluation of optical properties, a sample for an evaluation of sensory, and a foundation of Example 3 were produced, respectively.

### [Example 4]

### (Production of Titanium Oxide Powder)

A titanium oxide particle suspension (C3) was obtained in the same manner as in Example 1, except that by using the autoclave, the slurry (B1) was held at 130°C for 6 hours, then heated to 260°C, and held at the temperature of 260°C for 6 hours.

35% hydrochloric acid was added dropwise to the obtained titanium oxide particle suspension (C3) until the pH reached 7 in the same manner as in Example 2, and cleaning with pure water was repeated until the conductivity of a filtrate was 100 µS/cm.

Next, in the same manner as in Example 2, a solid matter on filter paper was collected, dried at 200°C, calcined at 700°C, and subjected to cracking to obtain a titanium oxide powder of Example 4.

### (Production of Cosmetics and the like)

In the same manner as in Example 1, a sample for an evaluation of optical properties, a sample for an evaluation of sensory, and a foundation of Example 4 were produced, respectively.

### [Example 5]

A titanium oxide powder of Example 5 was obtained in the same manner as in Example 2, except that the calcination was carried out at 800°C instead of the calcination at 700°C in Example 2.

### (Production of Cosmetics and the like)

In the same manner as in Example 1, a sample for an evaluation of optical properties, a sample for an evaluation of sensory, and a foundation of Example 5 were produced, respectively.

### [Comparative Example 1]

### (Production of Titanium Oxide Powder)

1 L of pure water was placed in a glass container having a capacity of 2 L, and 1 mol of tetraisopropoxytitanium (trade name: A-1, manufactured by Nippon Soda Co., Ltd.) was added dropwise while performing stirring to obtain a white suspension containing a hydrolyzed product of a titanium alkoxide.

Next, the white suspension was subjected to filtration, to obtain a white cake (X) that is a solid portion of the hydrolyzed product of titanium alkoxide.

Next, with respect to 1 mol of titanium oxide in the white cake, pyrrolidine (manufactured by Kanto Chemical Co., Inc.) in an amount of 0.7 mol and the white cake (X) were placed to an autoclave, and pure water was added therein to prepare a slurry (B11) in a total amount of 1 kg.

Next, by using the autoclave, the slurry (B11) was held at 220°C for 9 hours to obtain a reaction solution (C11) containing the titanium oxide particles.

Next, 100 g of the above described white suspension (C11) (containing 8 g of titanium oxide), 1 mol (80 g) of the white cake (X) in terms of titanium oxide, pyrrolidine (manufactured by Kanto Chemical Co., Inc.) in an amount of 0.7 mol, and pure water were added to an autoclave to prepare a slurry (B12) in a total amount of 1 kg.

Next, by using the autoclave, the slurry (B12) was held at 220°C for 9 hours to obtain a titanium oxide particle suspension (C12).

The obtained titanium oxide particle suspension (C12) was subjected to solid-liquid separation, and the solid was dried at 200°C to obtain a titanium oxide powder of Comparative Example 1.

### (Production of Cosmetics and the like)

In the same manner as in Example 1, a sample for an evaluation of optical properties, a sample for an evaluation of sensory, and a foundation of Comparative Example 1 were produced, respectively.

### [Comparative Example 2]

### (Production of Titanium Oxide Powder)

Next, 100 g of the reaction solution (C12) containing the titanium oxide particles that are obtained in the production process of Comparative Example 1 (containing 8.8 g of titanium oxide), 1 mol (80 g) of the white cake (X) in terms of titanium oxide, pyrrolidine (manufactured by Kanto Chemical Co., Inc.) in an amount of 0.7 mol, and pure water were added to an autoclave to prepare a slurry (B13) in a total amount of 1 kg.

Next, by using the autoclave, the slurry (B13) was held at 220°C for 9 hours to obtain a titanium oxide particle suspension (C13).

The obtained titanium oxide particle suspension (C13) was subjected to solid-liquid separation, and the solid was dried at 200°C to obtain a titanium oxide powder of Comparative Example 2.

### (Production of Cosmetics and the like)

In the same manner as in Example 1, a sample for an evaluation of optical properties, a sample for an evaluation of sensory, and a foundation of Comparative Example 2 were produced, respectively.

### [Comparative Example 3]

As Comparative Example 3, an anatase-type titanium oxide powder having a specific surface area of 7.0 m²/g (model number: ST-K4, manufactured by Sumitomo Osaka Cement Co., Ltd.) was used.

### (Production of Cosmetics and the like)

In the same manner as in Example 1, a sample for an evaluation of optical properties, a sample for an evaluation of sensory, and a foundation of Comparative Example 3 were produced, respectively.

### [Evaluation]

### (Measurement of BET Specific Surface Area)

A BET specific surface area of the titanium oxide powder of each of Example 1 to Example 5, and Comparative Example 1 to Comparative Example 3 was measured using a specific surface area meter (trade name: BELSORP-mini, manufactured by Bel Japan, Inc.). The results are illustrated in Table 1.

### (Crystallizability)

Crystallizability of each of the titanium oxide powders of Examples 1 to 5 and Comparative Examples 1 to 3 was confirmed with a field emission transmission electron microscope (FE-TEM) (trade name: JEM-2100F, manufactured by JEOL Ltd.). That is, whether titanium oxide particles are single-crystalline titanium oxide particles or polycrystalline titanium oxide particles was confirmed. The results are illustrated in Table 1.

### (Measurement of Particle Size Distribution)

Primary particle diameters of 50 titanium oxide particles contained in each of Examples 1 to 5 and Comparative Examples 1 to 3 were observed with a scanning electron microscope (SEM) (trade name: JSM-7200F, manufactured by JEOL Ltd.), respectively. Each of the obtained primary particle diameters was cubed and multiplied by a constant of 0.145 to calculate a volume of each of the titanium oxide particles. A volume particle size distribution was calculated using each primary particle diameter and a volume value thereof. A primary particle diameter (d50) in a case where the obtained volume particle size distribution represented by a cumulative volume percentage was 10% and a primary particle diameter (d50) in a case where the obtained volume particle size distribution represented by a cumulative volume percentage was 50% were calculated to calculate d10/d50. The results are illustrated in Table 1.

### (Amount of Titanium Oxide)

The amount of titanium oxide in the titanium oxide powder of each of Examples 1 to 5 and Comparative Examples 1 to 3 was measured in accordance with "53. Titanium dioxide quantification method " described in Japanese Standards of Quasi-Drug Ingredients 2006 (JSQI). The results are illustrated in Table 1.

### (Identification of Crystalline Phase of Titanium Oxide Particle)

The crystalline phase of the titanium oxide powder of each of Examples 1 to 5 and Comparative Examples 1 to 3 was identified using an X-ray diffractometer (trade name: X'Pert Pro, manufactured bySpectris Co., Ltd.). The results are illustrated in Table 1.

### (Measurement of Crystallinity of Titanium Oxide Powder)

The crystallinity of the titanium oxide powder of each of Example 1 to Example 5, and Comparative Example 1 to Comparative Example 3 was measured as follows. X-ray intensity was measured in an output of 45 kV and 40 mA at a diffraction angle 2θ in a range of 20° to 30° using CuKα ray as an X-ray source with an X-ray diffractometer (trade name: X'Pert PRO MPS, manufactured by Malvern Panalytical Ltd.). The obtained X-ray diffraction pattern was profile-fitted for a crystalline portion (peak) and an amorphous portion (halo), and integrated intensities of the portions were calculated, respectively. Next, a ratio of the integrated intensities of the crystalline portion to a total integrated intensity was defined as a crystallinity. The results are illustrated in Table 1.

### (Evaluation of L* Value)

The L* value of the titanium oxide powder of each of Examples 1 to 5 and Comparative Examples 1 to 3 was measured using a spectroscopic variable angle color difference meter (trade name: GC5000, manufactured by Nippon Denshoku Kogyo Co., Ltd.). The results are illustrated in Table 1.

### (Measurement of Average Friction Coefficient)

An average friction coefficient of the titanium oxide powder of each of Example 1 to Example 5, and Comparative Example 1 to Comparative Example 3 was measured according to the following procedure. 1 g of titanium oxide powder was placed on a skin model substrate (cheek skin model 30s (ϕ55 mm × 5 Tmm), manufactured by Beaulax Co., Ltd.).

Next, the skin model substrate on which 1 g of the titanium oxide powder was placed was disposed below a 1 cm² piano wire sensor (device standard) of a friction sensitivity tester (model number: KES-SE, manufactured by Kato Tech Co., Ltd.).

Next, the average friction coefficient was measured by moving the substrate horizontally by 30 mm at a speed of 1 mm/s in a state in which the sensor was brought into contact with the titanium oxide powder so as to have a load of 25 g (0.245 N). The results are illustrated in Table 1.

### (Evaluation of Average Secondary Particle Diameter)

The average secondary particle diameter of the titanium oxide powder of each of Examples 1 to 5 and Comparative Examples 1 to 3 was measured with a particle size distribution measuring device MASTERSIZER 3000 (manufactured by Malvern Panalytical Ltd.). That is, the volume particle size distribution d50 was obtained by dry measurement. The results are illustrated in Table 1.

### (Evaluation of Paleness, Feeling of Transparency, and Opacifying Power)

Each of the samples for an evaluation of optical properties of Examples 1 to 5, and Comparative Examples 1 to 3 was applied onto a 5 cm square substrate (trade name: HELIOPLATE HD-6, manufactured by Helioscreen) so as to be 12 mg to 14 mg to produce coated substrates.

A diffusion transmission spectrum (TT), a diffusion reflectionspectrum (TR), and a linear reflection spectrum (R) of the coated substrate were measured with a spectrophotometer (model number: UV-3150, manufactured by Shimadzu Corporation) and evaluated using the following indices. In all of measurements, a direction in which light is incident was measured from the coated surface, and reflection spectrum was measured on the basis of a molded plate obtained by compressing barium sulfate powder (special grade, manufactured by Kanto Chemical Co., Inc.).

The results are illustrated in Table 1.

### (Paleness)

A ratio (TR_{450 nm}/TR_{550 nm}) of the diffuse reflectance at 450 nm (TR_{450 nm}) to the diffuse reflectance at 550 nm (TR_{550 nm}) was used as an index of paleness. It can be said that as the ratio is larger than 1, paleness increases. Therefore, the smaller the value of TR_{450 nm}/TR_{550 nm}, the more preferable.

The correlation between indices of paleness and the appearance of a person is illustrated in Table 2.

### (Feeling of Transparency)

The ratio (R₅₅₀ₙₘ/TR₅₅₀ₙₘ) of the linear reflectance at 550nm (R₅₅₀ₙₘ) to the diffuse reflectance at 550nm (TR₅₅₀ₙₘ) was used as an index of a feeling of transparency. The smaller the ratio, the higher the feeling of transparency. Therefore, the smaller the value, the more preferable.

The correlation between indices of a feeling of transparency and the appearance of a person is illustrated in Table 2.

### (Opacifying Power)

The diffuse reflectance (TR₅₅₀ₙₘ) at 550 nm was used as an index for opacifying power. In a case where the diffuse reflectance is large, it can be said that the opacifying power is large. Thus, it is preferable that the value is large.

The correlation between indices of opacifying power and the appearance of a person is illustrated in Table 2.

**[Table 1]**

| | | Example 1 | Example 2 | Example 3 | Example 4 | Example 5 | Comparative Example 1 | Comparative Example 2 | Comparative Example 3 |
|---|---|---|---|---|---|---|---|---|---|
| E v a 1 u a t i o n | BET specific surface area (m²/g) | 9.0 | 9.0 | 9.0 | 8.0 | 8.8 | 9.0 | 6.0 | 7.0 |
| | Crystallizability | Single-crystalline | Single-crystalline | Single-crystalline | Single-crystalline | Single-crystalline | Single-crystalline | Single-crystalline | Polycrystal line |
| | d50 (nm) | 470 | 470 | 470 | 550 | 480 | 450 | 750 | - |
| | d10/d50 | 0.4 | 0.5 | 0.6 | 0.5 | 0.5 | 0.15 | 0.1 | - |
| | Amount of titanium oxide (%) | 99.0 | 99.5 | 99.5 | 99.5 | 99.5 | 99.0 | 99.0 | 99.5 |
| | Crystalline phase | Anatase single phase (anatase-type) | Anatase single phase (anatase-type) | Anatase single phase (anatase-type) | Anatase single phase (anatase-type) | Anatase single phase (anatase-type) | Anatase single phase (anatase-type) | Anatase single phase (anatase-type) | Anatase single phase (anatase-type) |
| | Crystallinity | 0.98 | 0.99 | 0.99 | 0.99 | 0.99 | 0.92 | 0.93 | 0.90 |
| | L* value | 70 | 91 | 93 | 93 | 91 | 70 | 70 | 93 |
| | Average friction coefficient | 0.3 | 0.3 | 0.3 | 0.2 | 0.3 | 0.6 | 0.6 | 0.2 |
| | Average particle diameter of secondary particles (µm) | 30 | 6 | 6 | 6 | 7 | 20 | 20 | 5 |
| | Paleness (TR₄₅₀ₙₘ/TR₅₅₀ₙₘ) | 1.06 | 1.06 | 1.06 | 1.05 | 1.06 | 1.06 | 1.05 | 1.09 |
| | Feeling of transparency (R₅₅₀ₙₘ/TR₅₅₀ₙₘ) | 0.01 | 0.01 | 0.01 | 0.01 | 0.02 | 0.01 | 0.02 | 0.02 |
| | Opacifying power (TR₅₅₀ₙₘ) | 60 | 60 | 59 | 63 | 60 | 50 | 53 | 49 |
| | Texture | × | ∘ | ∘ | ∘ | ∘ | × | × | ∘ |
| | Mean square displacement value | 6.0×10⁻⁵ | 1.0×10⁻⁶ | 2.0×10⁻⁶ | 3.0×10⁻⁶ | 1.0×10⁻⁶ | 6.0×10⁻⁵ | 6.0×10⁻⁵ | 6.0×10⁻⁵ |

**[Table 2]**

| | Value | Visual observation by person |
|---|---|---|
| Paleness TR₄₅₀ₙₘ/TR₅₅₀ₙₘ | Lower than 1.05 | None paleness |
| | 1.05 or higher and lower than 1.10 | Small paleness |
| | 1.10 or higher | Paleness |
| Feeling of transparency R₅₅₀ₙₘ/TR₅₅₀ₙₘ | Lower than 0.03 | There is feeling of transparency |
| | 0.03 or higher and lower than 0.05 | There is not much feeling of transparency |
| | 0.05 or higher | There is no feeling of transparency |
| Opacifying power TR₅₅₀ₙₘ | 45 or higher | There is opacifying power |
| | 40 or higher and lower than 45 | There is not much opacifying power |
| | Lower than 40 | There is no opacifying power |

### (Evaluation of Spreadability)

Each of the samples for an evaluation of sensory of Examples 1 to 5 and Comparative Example 1 was applied to the skin, and whether or not the samples for an evaluation of sensory of Examples 1 to 5 had better spreadability than Comparative Example 1 was evaluated by 10 people. As a result, all of the 10 people evaluated that the samples for an evaluation of sensory of Examples 1 to 5 had better spreadability than the sample for an evaluation of sensory of Comparative Example 1.

### (Evaluation of Texture)

The samples for an evaluation of sensory of Examples 1 to 5 and Comparative Examples 1 to 3 were applied to human skin. A case where a person who has the skin to which the samples were applied and feels that there was no roughness and the texture was good was evaluated as "∘", and a case where the person who has the skin to which the samples were applied and feels that there was roughness and the texture was bad was evaluated as "×". "∘" indicates that the evaluation is good, and "×" indicates that the evaluation is bad.

### (Evaluation of Mean Square Displacement Value)

Each of the foundations of the titanium oxide powders of Examples 1 to 5 and Comparative Examples 1 to 3 was applied to a glass substrate by screen printing to a thickness of about 20 µm. Leaving it to stand for 3 minutes, the glass substrate on which the foundation is applied was dried with a hot plate at 200°C for 5 minutes and used as a measurement sample 1 having a thickness of about 10 µm.

A reflectance (reflectance (1)) of the measurement sample 1 in a wavelength of 400 nm to 800 nm was measured with an integrating sphere using a spectrophotometer (trade name: V-700, manufactured by Nippon Spectroscopy Co., Ltd.).

Next, a black spacer having a thickness of 9 mm was provided on a surface coated with the measurement sample 1 in addition to the measurement point to obtain a measurement sample 2.

Similar to the measurement sample 1, a reflectance (reflectance (2)) of the measurement sample 2 in a wavelength of 400 nm to 800 nm was measured with an integrating sphere.

The reflectance (1) - the reflectance (2) was calculated with respect to the reflectance at a wavelength of 400 nm to 800 nm, and a high-angular reflectance (reflectance (3)) was calculated.

Next, the reflectance (3) ÷ the reflectance (1) was calculated at a wavelength of 400 nm to 800 nm, and a ratio of a high-angular scattering reflectance to an omnidirectional reflectance (reflectance (3)/reflectance (1)) was calculated.

Next, an arithmetic mean value (arithmetic mean value (4)) of the ratio of the high-angular scattering reflectance in a wavelength range of 400 nm to 800 nm (reflectance (3)/reflectance (1)) was calculated.

Next, the ratio of the high-angular scattering reflectance to the omnidirectional reflectance (reflectance (3)/reflectance (1)) was divided by the arithmetic mean value (4) ((reflectance (3)/reflectance (1)/arithmetic mean value (4)), and a standard value (hereinafter, referred to as "standard value (5)") at each wavelength was obtained.
(standard value (5) - 1)² was calculated at each wavelength, and a square displacement (square displacement (6)) was calculated at each wavelength.

Next, the square displacement (6) at a wavelength of 400 nm to 800 nm was arithmetically averaged, and a mean square displacement value (mean square displacement value (7)) was calculated.

The results are illustrated in Table 1.

### (Evaluation of Angle Dependence)

Each of the foundations of Examples 2 to 5 and the foundation of Comparative Example 1 was applied to the face, and the color differences between applied colors in a case of being observed from the front and observed from an oblique angle of 45 degrees were visually evaluated.

As a result, the foundations of Examples 2 to 5 looked the same even though the applied colors were observed from the front and observed from an oblique angle of 45 degrees.

On the other hand, the foundation of Comparative Example 1 had a bluish color applied and a poor complexion in a case of being observed from an oblique angle of 45 degrees as compared with the case of being observed from the front.

By the comparison of Examples 1 to 5 with Comparative Examples 1 to 3, it was confirmed that the titanium oxide powder which has the BET specific surface area of 5 m²/g or higher and 15 m²/g or lower, is formed of the single-crystalline titanium oxide particles, has d10/d50 of 0.3 or higher and 1 or lower, has the amount of titanium oxide of 99.0% by mass or more, and has an anatase-type crystalline phase can have both the opacifying power and the feeling of transparency, and reduce the paleness peculiar to the titanium oxide particles. It was also confirmed that the titanium oxide powder which has an average friction coefficient of 0.5 or lower and an average secondary particle diameter of 10 µm or lower has good spreadability and texture in a case where the cosmetics in which the titanium oxide powder is blended is applied to the skin. It was also confirmed that the titanium oxide powder having an L* value of 90 or higher had a small mean square displacement value and a small angle dependence of color appearance.

### Industrial Applicability

In the present invention, it is possible to provide the titanium oxide powder having excellent opacifying power in a case of being applied to the skin, and the dispersion and the cosmetics using the same.

Since the titanium oxide powder of the present invention has a BET specific surface area of 5 m²/g or higher and 15 m²/g or lower, is formed of the single-crystalline titanium oxide particles, has d10/d50 of 0.3 or higher and 1 or lower, has the amount of titanium oxide of 99.0% by mass or more, and has an anatase-type crystalline phase, both the opacifying power and the feeling of transparency can be achieved, and the paleness peculiar to the titanium oxide particles can be reduced, in a case of being applied to the skin. Therefore, the titanium oxide powder can be suitably used for base makeup cosmetics such as foundation. In addition, since the titanium oxide powder of the present invention has excellent performance as a white pigment, the titanium oxide powder can be used for industrial applications such as white ink, which is thus industrially valuable.

### Reference Signs List

- 10: Titanium oxide powder
- 100: Friction sensitivity tester
- 200: Stage
- 300: Skin model substrate
- 400: Piano wire sensor
- 500: Detector

## Claims

1. A titanium oxide powder, wherein
the titanium oxide powder has a BET specific surface area of 5 m²/g or higher and 15 m²/g or lower, and
the titanium oxide powder comprises single-crystalline titanium oxide particles,
wherein a value (d10/d50) that is obtained by dividing a value (d10), which is obtained when a particle size distribution represented by a cumulative volume percentage of primary particle diameters of the titanium oxide particles is 10%, by a value (d50), which is obtained when a particle size distribution represented by a cumulative volume percentage of the titanium oxide particles is 50%, is 0.3 or higher and 1 or lower,
an amount of titanium oxide thereof is 99.0% by mass or more, and
the titanium oxide powder has an anatase-type crystalline phase.

2. The titanium oxide powder according to claim 1,
wherein crystallinity thereof is 0.95 or higher.

3. The titanium oxide powder according to claim 1 or 2, wherein a value of L* in L*a*b* color space thereof is 90 or higher.

4. The titanium oxide powder according to any one of claims 1 to 3, wherein an average friction coefficient thereof which is measured at 0.245 N per 1 cm² is 0.5 or lower.

5. The titanium oxide powder according to any one of claims 1 to 4, wherein the titanium oxide powder has a mean square displacement value of 5.0 × 10⁻⁵ or lower, which is calculated by an evaluation method described below, (evaluation method)
the evaluation method comprises:
preparing cosmetics that contains the titanium oxide powder and a pigment;
measuring an omnidirectional reflectance (1) of the cosmetics in a wavelength range of 400 nm to 800 nm and a high-angular reflectance (3) of the cosmetics in a wavelength range of 400 nm to 800 nm;
calculating a value (reflectance (3)/reflectance (1)) that is obtained by dividing the high-angular reflectance (3) at each wavelength by the omnidirectional reflectance (1) at each wavelength, respectively;
calculating an arithmetic mean value (4) of the values (reflectance (3)/reflectance (1)), which are obtained by the division in a range of 400 nm to 800 nm;
calculating a standard value (5) at each wavelength by dividing the value (reflectance (3)/reflectance (1)), which is obtained by the division at each wavelength, by the arithmetic mean value (4); and
calculating a value of a mean square displacement of the standard value (5) and 1.

6. The titanium oxide powder according to any one of claims 1 to 5, wherein an average secondary particle diameter is 1 µm or higher and 10 µm or lower.

7. The titanium oxide powder according to any one of claims 1 to 6, wherein the titanium oxide powder has an inorganic compound or an organic compound on a surface thereof.

8. A dispersion comprising:
the titanium oxide powder according to any one of claims 1 to 7; and
a dispersion medium.

9. Cosmetics comprising:
the titanium oxide powder according to any one of claims 1 to 7; and
a cosmetic base.

10. Cosmetics comprising:
the dispersion according to claim 8; and
a cosmetic base.
